**Europäisches Patentamt**

(19) **European Patent Office**

**Office européen des brevets**

(11) Publication number: **0 058 077**
**A2**

(12) **EUROPEAN PATENT APPLICATION**

(21) Application number: **82300615.0**

(22) Date of filing: **08.02.82**

(51) Int. Cl.³: **A 61 F 1/20**

(30) Priority: **09.02.81 US 232941**

(43) Date of publication of application:
**18.08.82 Bulletin 82/33**

(84) Designated Contracting States:
**DE FR GB IT NL SE**

(71) Applicant: Fredrickson, John Murray
24 Queen Mary's Drive
Toronto(CA)

(72) Inventor: Fredrickson, John Murray
24 Queen Mary's Drive
Toronto(CA)

(74) Representative: Howden, Christopher Andrew et al,
FORRESTER & BOEHMERT Widenmayerstrasse 4/1
D-8000 München 22(DE)

(54) Implantable artificial sound source.

(57) An implantable sound source for human voice production to replace the natural larynx surgically removed, for example, because of cancer, consists of a disc-like stainless steel body (10) one circular wall of which consists of a diaphragm (14) laser-welded to the remainder. Circumferential suture-receiving bores (18) are provided for fastening the source to the host body. Apiezoceramic transducer (22) is cemented to the diaphragm interior wall and is fed via an electromagnetic coupling consisting of a secondary coil (30) implanted in the chest wall of the host. A coupling core (36) is in two parts spring-biased together and carries a primary coil (38) connected to an AC electric power source, thus permitting the core to be disconnected at will leaving only the implanted portions. The frequency of the supply is allowed to vary somewhat to give a more natural-sounding reproduction and, for the same purpose, is arranged to "droop" over a short period corresponding approximately to the length of a normal breath.

Croydon Printing Company Ltd.

Title: "Implantable Artificial Sound Source"

The present invention is concerned with improvements in or relating to artificial sound sources for human voice production, and especially to such a device that can be surgically implanted in the throat of the user.

The treatment of carcinoma of the larynx frequently involves laryngectomy (removal of the larynx) with consequent loss of speech. Approximately 50% of the patients treated in this way are able to develop the so-called esophageal speech, but the remaining 50% are unsuccessful with consequent practical and pyschological problems. At the present time approximately 3000 laryngectomies are performed each year in North America alone. Surgical reconstructive procedures which enable the pulmonary system to be used as a power source for speech have not so far proven successful in the long term, since they usually involve production of an internal fistula to provide the necessary pulmono-pharyngeal connection, with subsequent inherent problems of stenosis and/or aspiration.

A number of relatively successful proposals have been made for externally-applied sound sources which are held against the user's neck when speech is desired, the user's throat thus being used to modulate the signal provided by the source with the speech issuing from the user's mouth. Unfortunately these units are not only cumbersome and conspicuous in use but the speech sound is monotonous, hard to understand and at times irritating to the listener. A basic problem is that only a small percentage of the available acoustic power is transferred transcutaneously and most of it is lost, even if the device is held up tight against the skin; the use of a coupling liquid or cream would improve the transfer but is impractical for daily use.

In the present invention, there is provided an implantable sound source, which generates a sound in the throat cavity of the host, in response to externally supplied power. The host can then use intact throat, mouth, etc. muscles to modulate the sound, and produce a simulated speech issuing from the lips of the host. In this way, the host can use lips in the normal way, within sight of the listener, so that the comprehensibility of the simulated speech issuing from the lips in enhanced. The sound source comprises a body member with a vibratable diaphragm and a transducer to which power is supplied from an external source to cause diaphragm vibration. The device is unobtrusively implanted, and the external parts are readily disconnected and removable if desired.

Thus the present invention provides an artificial sound generating apparatus for use by a living host in the production of simulated speech, said apparatus comprising in combination, a sound source, an electrical power source for said sound source and coupling means for electrically coupling the sound source and the power source, characterised in that: the sound source is implantable adjacent to the throat cavity of the host and comprises a body member, a vibratable diaphragm forming an enclosure with said body member and an electrical-acoustic transducer mounted in the enclosure and connected to the diaphragm to produce vibration thereof upon supply of electrical power from said power source, the power source is disposed exteriorly of the host's body and has external electrical leads, and the coupling means electrically connects the external electrical leads of the power source to the internal leads from the transducer.

A particular preferred embodiment of the invention will now be described, by way of example, with reference to the accompanying diagrammatic drawing wherein:

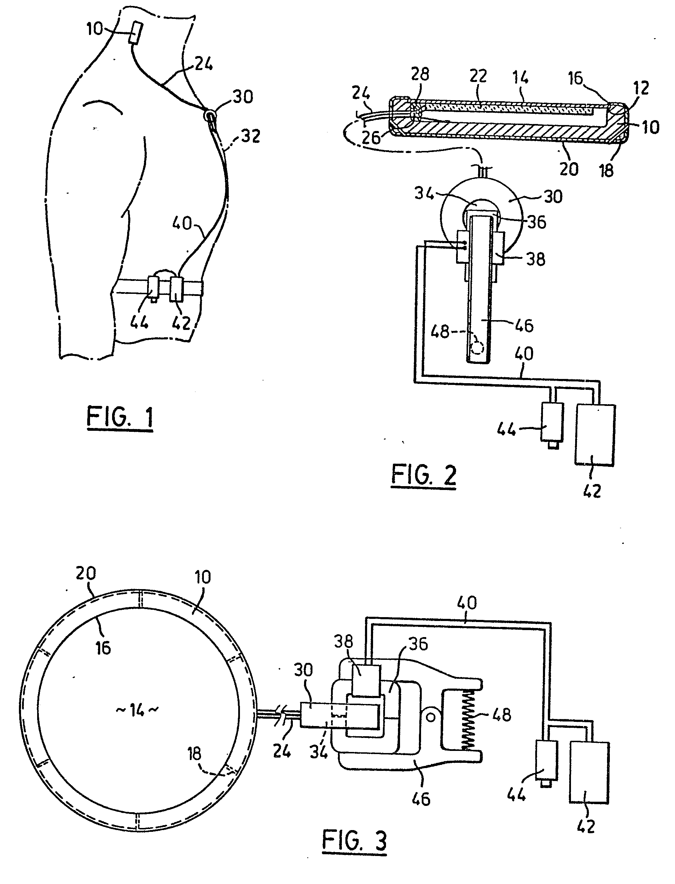

FIGURE 1 is a part section through a human torso to show the relative location of the necessary implants and the connected apparatus,

FIGURE 2 is a transverse cross-section through the acoustic generator, but with the accompanying wiring and coupling member shown diagrammatically; and

FIGURE 3 is a plan view of the acoustic generator and the coupling member of Figure 2.

The particular embodiment shown in the drawings consists of a hollow body member 10 of circular shape as seen in plan having one circular side open, the open side having a larger diameter shallow counterbore providing an annular ledge 12. A thin circular diaphragm 14 fits closely within the counterbore on the ledge and is fastened therein by an accurate circular laser weld 16 that at the same time completely seals this particular pathway against the passage of body fluids, etc. The body 10 is provided with a plurality of circumferentially-spaced suture-receiving bores 18, by which it is fastened into place in the host body at least until it has been overgrown by sufficient surrounding tissue for support thereby. The exterior of the casing is provided with a coating 20 of a material which will facilitate this tissue attachment, such as a biologically compatible cobalt chromium-molybdenum alloy applied in powder form.

The diaphragm 14 is driven to produce the required acoustic output by a piezoceramic transducer 22 cemented to the inner surface thereof, electric power being supplied to the transducer via leads 24 that pass through the body wall. A strain relief member 26 is embedded in a blob 28 of epoxy cement that also seals the casing interior. The body member is implanted surgically behind the throat cavity in the retropharyngeal space of the host while the internal leads 24 are lead through the body to terminate at a hollow, angular secondary induction coil 30.

This coil is surgically implanted on the chest wall 32 in a skin tunnel formed surgically in the known manner so that the coil 30 and its central bore 34 are totally skin covered but project from the chest wall 32 to provide external accessibility of the skin-line central bore 34. Into the bore 34 is positioned a removable ferrite core 36 carrying a primary induction coil 38 that is connected via external leads 40 to a power source 42 consisting of a rechargeable secondary battery, and an interposed control switch 44 and the required electrical circuitry.

The core 36 is formed in two mating parts mounted on the arms of a spring clip member 46, the compression spring 48 of which holds the parts firmly in contact with one another to ensure continuity of the magnetic circuit. At any time the core 36 can be removed from its position within the skin tunnel lining central bore 34, so that the external components can be disconnected from the host body, for example during bathing, sleep, etc., leaving only the implanted elements 10, 24 and 30.

In this specific embodiment the body 10 is 4.0 cm diameter and 6.5 mm thick and weighs about 23.5 grams, the material used being 316L stainless steel. The coil 30, that is embedded is enclosed in medical grade epoxy cement. The electrical power required for adequate acoustic output is surprisingly high and the piezoceramic transducer used produced 85-95 dB (linear) at a distance of 30 cm in free air measured on axis. The battery 42 must be able to supply about 4 watts of power for a reasonably continuous period of use, such as 3-4 hours, which is sufficient for one day's conversation.

In an alternative arrangement, to avoid the formation of a skin tunnel used on the chest wall 32, a flat secondary induction coil may be used, and implanted immediately below the skin. Then a flat primary induction coil may be removably affixed, e.g. by adhesive, to the exterior of the skin immediately over the implanted secondary coil, to provide the necessary transcutaneous power connection. Other means of transcutaneous power connection, such as capacitive coupling, may also be adopted.

The power supply provides the electrical power at a frequency to provide an acoustic output at the necessary frequency for human speech, and also to provide the necessary inductive coupling through the coil 30. From this acoustic output, the host makes simulated speech by use of throat, mouth and lip muscles, in the same manner as in production of natural speech. A suitable frequency for the output is about 97 hertz; to avoid a monotonous effect it is desirable not to hold the frequency constant, but instead to allow it to vary somewhat, for example $\pm$ 3 hertz, to give a variation to the resultant speech more nearly simulating the natural frequency variation of the human voice. A flat frequency response over the approximate range 50 - 5000 CPS should be arranged.

For the same purpose the power supply may also be arranged to vary the output frequency progressively and continuously over a short period of time in dependence upon the speech patterns of the host. This may be achieved by equipping the power source and its associated control circuitry with a program to vary the frequency of the input to the device, thereby varying the pitch and intonation of the sound produced in response, by the implanted source. For example, if, as is usual with English-speakers, the frequency tends to decrease with time over the period of a single breath, then the frequency of the power source is arranged to drop with the same effect over the time period that is occuplied by a normal breath. If the frequency tends to increase then the reverse effect would be arranged.

In another embodiment, a myoelectric (muscle operated) activation source is used to operate the switch and circuitry of the device. Then the host may supply power to and control the device even less obtrusively. Also, the transducer-diaphragm combination may be formed by an electro-responsive polymeric membrane, e.g. polyvinylfluoridine, which responds audibly to electric current applied thereto.

CLAIMS

1.      An artificial sound generating apparatus for use by a living host in the production of simulated speech, said apparatus comprising in combination, a sound source, an electrical power source for said sound source and coupling means for electrically coupling the sound source and the power source, characterised in that the sound source is implantable adjacent to the throat cavity of the host and comprises a body member (10), a vibratable diaphragm (14) forming an enclosure with said body member and an electrical-acoustic transducer (22) mounted in the enclosure and connected to the diaphragm to produce vibration thereof upon supply of electrical power from said power source, the power source (42) is disposed exteriorly of the host's body and has external electrical leads (40), and the coupling means (30, 36, 38) electrically connects the external electrical leads of the power source to the internal leads (24) from the transducer.

2.      An implantable sound source as claimed in claim 1, wherein the said body member and the diaphragm are of metal and are laser welded to one another around the periphery of the diaphragm.

3.      An implantable sound source as claimed in claim 1 or 2, wherein the exterior of the said body is provided with a coating for the promotion of tissue growth thereon.

4.      An implantable sound source as claimed in any one of claims 1 to 3, wherein the said body is provided with a plurality of circumferentially spaced suture-receiving bores (18).

5.      An implantable sound source as claimed in any one of claims 1 to 4, wherein coupling means comprise: a hollow electric secondary induction coil (30) implantable within the host body with the coil hollow (34) exposed, and a coupling core member (36) insertable through the thus implanted hollow coil and carrying a primary induction coil (38) for transfer of energy therefrom to the said secondary induction coil.

6.      An implantable sound source as claimed in claim 5, wherein the coupling core member is split and is releasable from the secondary induction coil.

7. An implantable sound source as claimed in claim 6, wherein the coupling core member comprises two separate parts spring urged into engagement with one another for insertion through the hollow secondary coil and release therefrom.

FIG. 1

FIG. 2

FIG. 3